# EUROPEAN PATENT SPECIFICATION

(11) **EP 0 738 505 B1**
(45) Date of publication and mention of the grant of the patent: **01.12.1999**
(21) Application number: 95830146.7
(22) Date of filing: 11.04.1995
(51) Int. Cl.: A61F 13/15

(54) **Method for the production of a laminated material impermeable to fluids**
Verfahren zur Herstellung eines flüssigkeitsdichten Schichtmaterials
Procédé pour la production d'un matériau laminé imperméable aux fluides

(43) Date of publication of application: 23.10.1996
(73) Proprietor: PANTEX S.r.l., I-51031 Agliana, Pistoia (IT)
(72) Inventor: Giacometti, Claudio, I-51030 Pieve a Celle, Pistoia (IT); BARGIACCHI, Franco, Pistoia/IT (IT)
(74) Representative: Mannucci, Gianfranco, Dott.-Ing.

(56) References cited:
- FR-A- 2 455 884
- US-A- 2 896 626
- US-A- 2 897 109
- US-A- 4 725 473

## Description

### Technical field

The invention relates to a process for the production, of an impermeable laminated material the said material being particularly intended for use in the manufacture of absorbent products such as baby diapers, sanitary napkins, incontinence pads and the like.

### State of the art

Sanitary napkins, baby diapers, incontinence pads and the like are currently produced using an impermeable backsheet, an intermediate absorbent mass and a topsheet that is permeable to fluids. The backsheet and the topsheet are joined along the edge of the product by a suitable method, such as welding, gluing or the like.

The topsheet must have a structure that permits fluid to pass rapidly from its external to its internal surface, i.e. into the absorbent mass. It must also prevent the fluid passing back to the outside, even when the absorbent mass is compressed, for example by the body weight of the user, and must enable a certain distance to be maintained between the skin of the user (which is in contact with the outer surface of the topsheet) and the absorbent mass inside. Finally, because the topsheet comes into contact with the skin, it is advisable that it should feel pleasant to the touch and have a textile handle.

By contrast, the backsheet must be impermeable to fluids. It normally comprises a simple plastic film. Absorbent products of this type are described for example in USA patents 4,781,962; 4,333,979; 4,761,322; 3,929,135; 3,814,101.

The absorbent product described in USA patent No. 4,781,962 has a permeable topsheet consisting of too thin sheets joined together: the first, outermost, thin sheet is made of textile fibres partially consolidated by hot lamination, while the second is a perforated plastic film. The textile fibre sheet has areas of increased density that coincide with the perforations, the increased density being achieved by compressing and heating the fibres of the sheet, and is anchored to the plastic film by partial fusion of the fibres around the hole. The perforation and the welding together of the two components are achieved by laminating the material between a heated roll bearing projections and a smooth roll. A further web like material for the production of an impervious backsheet is shown in FR-A-2,455,884, where a method according to the preamble of claim 1 is disclosed. In US-A-4,725,473 a method is disclosed according to which the unconsolidated non-woven web is bonded to the underlying film by means of discrete points of adhesion.

A further method for bonding a web of fibers to a film is disclosed in US-A-2,897,109.

### Objects of the invention

The main object of the invention is to produce a flexible and impermeable laminated material that is particularly suitable for the manufacture of absorbent products and that has better properties than conventional plastic films, particularly for use as a backsheet.

Another object of the present invention is the production of a laminated material that is extremely strong and at the same time flexible and soft while retaining the typical impermeable properties of plastic films.

A further object of the present invention is the production of a laminated material that permits the manufacture of an absorbent product with specific tactile properties, thereby making it more pleasant to use.

Further objects and advantages of the present invention will become evident to experts in the field on reading the text below.

### Summary of the invention

These objects are achieved with a method according to claim 1.

The method includes the following steps:
a) producing a continuous web of unconsolidated textile fibres;
b) aligning the said continuous web of unconsolidated textile fibres with a plastic film;
c) joining the said web of unconsolidated textile fibres and the said plastic film by the application of pressure and heat to the said web and the said plastic film. Joining is obtained along the entire surface of contact between the fiber web and the plastic film by applying a pressure and a temperature which are sufficient to produce partial fusion of the fibers of said web and consequently join said web and said film together.

An impermeable laminated material is thus produced that is suitable for use as a backsheet or back layer in an absorbent product. The presence of the textile fibres, applied in the form of an unconsolidated web, gives the strip laminated material a high degree of flexibility. In addition, applying the material to the back of the absorbent product so that the fibre-coated surface is on the exterior of the product eliminates direct contact between the epidermis and the plastic film, making the product more comfortable to use.

Embossing the laminated material comprising the plastic film and the fibre web during the joining process makes the material softer and more flexible, thereby making the absorbent product made with the said material even more comfortable to use.

The fibre web and the plastic film are joined by means of calendering or lamination, which involves the application of adequate pressure and heat, by two rolls. It is advantageous when selecting the materials for the fibres and the film to ensure that the fibres have a lower melting point than the film. This enables the fibres to be welded to the film without the risk of damage to the wholeness of the film, which would result in the loss of its impermeability properties.

### Brief description of the drawings

The invention will be better understood from the description and attached drawing, which shows a practical nonlimiting embodiment of the said invention. In the drawing:
Fig. 1 shows a diagram of a plant for the production of the laminated material according to the invention;
Fig. 2 shows a diagrammatic enlarged local section of the material according to the invention; and
Figs. 3 and 4 show two examples of products made with the laminated material.

### Detailed description of the invention

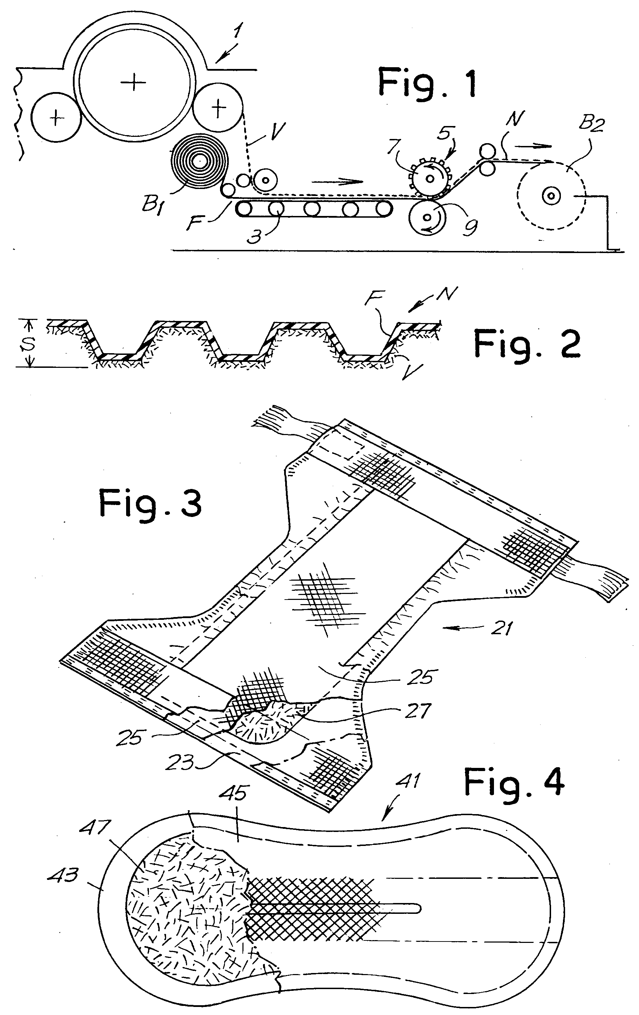

Fig. 1 shows a diagrammatic representation of a plant for manufacturing the product according to the invention. 1 generically indicates a carding machine which produces a web V of unconsolidated textile fibres. After it emerges from the carding machine the web V is aligned with a plastic film F unwound from a feed reel B1. A conveyor belt 3 supports and transports the web V and the film F to a calendering machine 5 comprising two counter-rotating rolls 7 and 9, whose directions of rotation are shown. The two rolls 7 and 9 are pressed against each other and at least one, or preferably both, is/are heated, for example by steam or another heat-transfer fluid circulating inside it/them, or by electricity or some other suitable means.

The pressure between the rolls 7 and 9 and their surface temperature are sufficient to produce partial fusion of the fibres and consequently join together, basically over the entire reciprocal contact surface, the web V and the film F and so obtain at the exit from the calendering machine 5 the composite strip laminated material N, which is wound onto a reel B2.

As illustrated diagrammatically in Fig. 1, the upper roll 7 has a series of projections to emboss the composite material passing between the two rolls. In this way the strip material N obtained is embossed over its entire surface or over part of its surface, depending on the distribution of the projections on the roll 7. The pressure between the rolls and the temperature are sufficient to produce permanent deformation of the plastic film and partial fusion of the fibres, thus welding together the two components of the strip material The surface of the opposing roll 9 can be made of pliable material to facilitate the embossing of the film F and the web V joined to it.

Alternatively, smooth rolls can also be used to join the two components without embossing them.

The plastic film used can be a polyethylene or modified polyethylene film or equivalent between 12 and 30 micrometres thick. The web V can be made of polyethylene fibres or derivatives, polypropylene fibres or derivatives, two-component fibres with a polypropylene or polyester core and a polyethylene coating, or mixtures of the said fibres. The web has a weight of between 10 and 25 g/m². After the embossing, a strip material N is obtained, the weight of which depends on the weight of the film and that of the fibre web but is typically between 30 and 50 g/m², and preferably around 40 g/m², with a typical thickness s of between 0.2 and 0.4 mm and preferably around 0.3 mm. The embossing can be effected with frustopyramidal projections or projections of another suitable shape, for example frustoconical. To make the strip material N sufficiently soft and strong, it is advantageous to use a typical projection density of between 30 and 100 projections/cm², and preferably between 40 and 70 projections/cm².

The film F advantageously has a higher melting point than the fibres so that in the calendering stage joining is achieved by means of fusion of the fibres without fusion of the film and without putting the wholeness of the said film at risk. To this end the temperature of the upper roll is advantageously between 150 and 180 °C, while the temperature of the lower roll is advantageously between 100 and 150 °C. The rate of feed of the film and of the web V from the carding machine is advantageously between 20 and 70 m/min.

Fig. 2 shows a greatly enlarged local section of the embossed strip material N produced by the calendering machine 5.

Fig. 3 shows a baby diaper 21, with a backsheet 23 made with a piece of strip material N of the type described above and a topsheet 25 that is permeable to fluids. The topsheet 25 can be a nonwoven fabric, a perforated plastic film or a multilayer product obtained by joining together one or more layers of nonwoven fabric and a plastic film. A layer of absorbent material 27 is placed between the backsheet 23 and the topsheet 25 and absorbs the fluid that passes through the topsheet 25.

Fig. 4 shows the use of the strip material N as the impermeable backsheet in a sanitary napkin 41. 43 indicate the backsheet made of impermeable plastic film joined to the web of textile fibres, 45 indicates the permeable topsheet (made of nonwoven fabric, perforated plastic film, a composite material or the like), and 47 indicates the absorbent mass inside.

The drawing is intended to show only one embodiment, given solely as a practical demonstration of the invention, which can vary in form and disposition without thereby departing from the scope of the underlying concept of the said invention. The presence of any reference numbers in the attached claims is intended to facilitate the reading of the claims with reference to the description and the drawings and does not limit the scope of protection afforded by the claims.

## Claims

1. Method for the production of a strip material that is impermeable to fluids, including the following steps:
a) producing a continuous web of unconsolidated textile fibres (V);
b) aligning the said continuous web of unconsolidated textile fibres (V) with a plastic film (F);
c) joining together said web of unconsolidated textile fibres (V) and said plastic film by the application of pressure and heat to said web and said plastic film, such that said web and said film are joined together basically along the entire contact surface thereof,
characterised in that the pressure and temperature applied are sufficient to produce partial fusion of the fibers of said web and consequently join said web and said film together.

2. Method according to Claim 1, characterized in that the said strip material is embossed during the process to join the web of unconsolidated textile fibres (V) and the plastic film (F).

3. Method according to claim 1 or 2, characterized in that said web is formed by synthetic thermoplastic fibers having a melting point lower than the melting point of said plastic film.

4. Method according to one or more of the preceding claims, characterized by the further step of producing an absorbent product (21; 41) comprising a backsheet (23; 43) that is impermeable to fluids, a topsheet (25; 45) that is permeable to fluids, a layer of absorbent material (27; 47) placed between the said backsheet (23; 43) and the said topsheet (25; 45), wherein said backsheet is formed by portion of said strip material.

## Patentansprüche

1. Verfahren für die Herstellung eines Streifenmaterials, das für Flüssigkeiten undurchlässig ist, mit den folgenden Schritten:
a) Herstellen einer kontinuierlichen Bahn von unverfestigten Textilfasern (V);
b) Ausrichten der kontinuierlichen Bahn von unverfestigten Textilfasern (V) mit einer Kunststoffolie (F);
c) Verbinden der Bahn aus unverfestigten Textilfasern (V) und der Kunststoffolie durch Anwendung von Druck und Wärme auf die Bahn und die Kunststoffolie derart, daß die Bahn und die Folie im wesentlichen auf ihrer gesamten Kontaktfläche miteinander verbunden werden;
dadurch **gekennzeichnet,** daß der aufgewendete Druck und die Temperatur ausreichend sind, um ein teilweises Schmelzen der Fasern der Bahn zu bewirken und dadurch die Bahn und die Folie miteinander zu verbinden.

2. Verfahren nach Anspruch 1, dadurch **gekennzeichnet,** daß während des Verfahrens zum Verbindens der Bahn von unverfestigten Textilfasern (V) und der Kunststoffolie (SF) das Streifenmaterial geprägt wird.

3. Verfahren nach Anspruch 1 oder 2, dadurch **gekennzeichnet**, daß die Bahn aus synthetischen thermoplastischen Fasern gebildet wird, deren Schmelzpunkt niedriger als der Schmelzpunkt der Kunststofffolie ist.

4. Verfahren nach einem oder mehreren der vorangehenden Ansprüche,
**gekennzeichnet** durch den weiteren Schritt des Herstellens eines absorbierenden Produktes (21, 41) mit einer Basislage (23, 43), die flüssigkeitsundurchlässig ist, einer Decklage (45; 45) die flüssigkeitsdurchlässig ist, und einer Schicht aus absorbierendem Material (27, 47) zwischen der Basislage (23, 43) und der Decklage (25, 45), wobei die Basislage aus einem Abschnitt des Streifenmaterials gebildet wird.

## Revendications

1. Procédé de production d'un matériau formant bande qui est imperméable aux fluides, comprenant les étapes suivantes :
a) fabrication d'une bande continue de fibres de textile non consolidées (V) ;
b) alignement de ladite bande continue de fibres de textile non consolidées (V) avec un film de plastique (F) ;
c) assemblage de ladite bande de fibres de textile non consolidées (V) et dudit film de plastique par l'application d'une pression et de chaleur à ladite bande et audit film de plastique de telle sorte que ladite bande et ledit film sont assemblés ensemble essentiellement sur la totalité de la surface de contact de ces derniers, caractérisé en ce que la pression et la température appliquées sont suffisantes pour assurer la fusion partielle des fibres de ladite bande, et par conséquent, assembler ladite bande et ledit film ensemble.

2. Procédé selon la revendication 1, caractérisé en ce que ledit matériau formant bande est gaufré pendant le procédé pour assembler la bande de fibres de textiles non consolidées (V) et le film de plastique (F).

3. Procédé selon la revendication 1 ou la revendication 2, caractérisé en ce que ladite bande est formée par des fibres thermoplastiques synthétiques dont le point de fusion est inférieur à celui dudit film de plastique.

4. Procédé selon l'une quelconque ou plusieurs des revendications précédentes, caractérisé par l'autre étape consistant à produire un produit absorbant (21 ; 41) comprenant une feuille arrière (23 ;43) qui est imperméable aux fluides, une feuille de dessus (25 ;45) qui est perméable aux fluides, une couche d'un matériau absorbant (27 ; 47) placée entre ladite feuille arrière (23 ; 43) et ladite feuille de dessus (25 ; 45) selon laquelle ladite feuille arrière est formée par une partie dudit matériau formant bande.
